(19) Europäisches Patentamt
European Patent Office
Office européen des brevets

(11) Veröffentlichungsnummer: **0 010 295**
**B1**

(12) # EUROPÄISCHE PATENTSCHRIFT

(45) Veröffentlichungstag der Patentschrift: **28.07.82**

(51) Int. Cl.³: **C 07 C 31/08,** C 07 C 29/15 // B01J23/46, B01J23/56, B01J23/64

(21) Anmeldenummer: **79104009.0**

(22) Anmeldetag: **17.10.79**

(54) **Verfahren zur Herstellung von Ethanol aus Synthesegas.**

(30) Priorität: **24.10.78 DE 2846148**

(43) Veröffentlichungstag der Anmeldung: **30.04.80 Patentblatt 80/9**

(45) Bekanntmachung des Hinweises auf die Patenterteilung: **28.07.82 Patentblatt 82/30**

(84) Benannte Vertragsstaaten: **BE DE FR GB IT NL**

(56) Entgegenhaltungen:
**DE-A-2 503 204**
**DE-A-2 628 576**
**DE-A-2 813 543**
**DE-C-871 889**
**GB-A-313 061**
**GB-A-337 409**

(73) Patentinhaber: **HOECHST AKTIENGESELLSCHAFT, Postfach 80 03 20, D-6230 Frankfurt/Main 80 (DE)**

(72) Erfinder: **Leupold, Erst Ingo, Dr., Am Zäunefeld 15, D-6392 Neu-Anspach (DE)**
Erfinder: **Schmidt, Hans-Joachim, Dr., Burgenblick 6, D-6240 Königstein/Taunus (DE)**
Erfinder: **Wunder, Friedrich, Dr., Jahnstrasse 46, D-6093 Flörsheim am Main (DE)**
Erfinder: **Arpe, Hans-Jürgen, Dr., Ridder-Weg 10, D-6230 Frankfurt am Main 80 (DE)**
Erfinder: **Hachenberg, Horst, Dr., Mohnweg 1, D-6229 Walluf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Ethanol aus Synthesegas

Die vorliegende Erfindung betrifft ein Verfahren zur Herstellung von Ethanol durch Umsetzung von Kohlenmonoxid mit Wasserstoff an einem Rhodium-Trägerkatalysator.

Es ist bereits aus den deutschen Auslegeschriften 2 503 233 und 2 628 463 bekannt, dass die Gasphasenumsetzung von Synthesegas an Rhodiummetall enthaltenden Katalysatoren im wesentlichen zu Gemischen sauerstoffhaltiger Produkte mit zwei Kohlenstoffatomen im Molekül, wie Essigsäure, Ethanol und Acetaldehyd führt.

.Aus der deutschen Auslegeschrift 2 503 204 ist weiterhin bekannt, dass die Selektivität durch Zusatz von Eisensalzen zugunsten von Ethanol beeinflusst wird. Der Zusatz von Eisensalzen führt jedoch gleichzeitig zu einer starken Verringerung der Aktivität des Rhodium-Katalysators. So sind nach Tabelle 1 der deutschen Auslegeschrift 2 503 204 die Raum-Zeit-Ausbeuten eisenhaltiger Rhodium-Katalysatoren etwa um den Faktor vier niedriger als die eines (zum Vergleich angegebenen) eisenfreien Rhodium-Katalysators. Ein derartiger Leistungsabfall in der Bildung sauerstoffhaltiger $C_2$-Produkte ist aber im Hinblick auf eine wirtschaftliche Nutzung äusserst unbefriedigend.

Weiterhin ist aus dem US-Patent 4 096 164 bekannt, dass durch Zusatz von Molybdän oder Wolfram zu Rhodium enthaltenden Katalysatoren die Selektivität der Bildung von Alkoholen allgemein erhöht wird. Beide Cokatalysatoren führen aber nicht zu einer wesentlich höheren Ethanolbildung, sondern hauptsächlich zu einer stärkeren Bildung von Methanol, Propanol und Butanol.

Somit bestand die Aufgabe, die Ethanol-Selektivität von Rhodium-Katalysatoren zu verbessern, d.h. die Bildung von anderen Produkten wie Methanol, Propanol und Butanol zu verringern, ohne gleichzeitig die Raum-Zeit-Ausbeute zu Ethanol herabzusetzen.

Es wurde nun gefunden, dass die Ethanol-Selektivität und zugleich die Ethanol-Raum-Zeit-Ausbeute durch Einsatz von Rhodium-Katalysatoren, die eines oder mehrere der Elemente Zirkon, Hafnium, Lanthan, Platin, Chrom und Quecksilber auf einem Träger enthalten, entscheidend verbessert werden kann.

In der nicht vorveröffentlichten europäischen Patentanmeldung EP-A1-0 004 656 wird zwar ein Verfahren zur Herstellung von Essigsäure, Ethanol, Acetaldehyd und gegebenenfalls ihren Folgeprodukten durch Umsetzung von Kohlenmonoxid mit Wasserstoff an Rhodiummetall enthaltenden Trägerkatalysatoren beschrieben, das dadurch gekennzeichnet ist, dass die Katalysatoren zusätzlich Halogenidionen und Magnesiumsalze und/oder -komplexverbindungen und/oder Verbindungen des Magnesiums mit Oxiden von Elementen der III. bis VI. Gruppe des periodischen Systems enthalten. Als Beispiele für die letztgenannte Klasse von Magnesiumverbindungen werden unter anderen Magnesiumzirkonate und Magnesiumchromite genannt. Überraschenderweise ist – falls man eines der Elemente Zirkon, Hafnium, Lanthan, Platin, Chrom oder Quecksilber einsetzt – die Gegenwart von Magnesium bei der Herstellung von Ethanol nicht notwendig, wenngleich keineswegs schädlich. Ausserdem haben sich bestimmte chlorhaltige Rhodiumkomplexe als geeignet erwiesen, die in der genannten europäischen Patentanmeldung nicht beschrieben werden.

Ein Gegenstand der Erfindung ist daher ein Verfahren zur Herstellung von Ethanol durch Umsetzung von Kohlenmonoxid mit Wasserstoff an einem Rhodium-Trägerkatalysator, das dadurch gekennzeichnet ist, dass der Trägerkatalysator eines oder mehrere der Elemente Zirkon, Hafnium, Lanthan, Platin, Chrom und Quecksilber in Form ihrer Salze oder Komplexverbindungen als Cokatalysator, aber im Falle von Zirkon und Chrom kein Magnesium enthält.

Ein weiterer Gegenstand der Erfindung ist ein Verfahren zur Herstellung von Ethanol durch Umsetzung von Kohlenmonoxid mit Wasserstoff an einem Rhodium-Trägerkatalysator, das dadurch gekennzeichnet ist, dass man Rhodium in Form chlorhaltiger Komplexe der allgemeinen Formel $Me_m[RhCl_6]_n$, wobei Me für eines der katalytisch wirkenden Elemente Zirkon, Hafnium, Lanthan, Platin, Chrom und Quecksilber steht, verwendet.

Der Befund, dass sich die Zusammensetzung des Reaktionsproduktes durch Verwendung der genannten Elemente zugunsten der Ethanolbildung bei gleichzeitiger Erhöhung der Raum-Zeit-Ausbeute verschiebt, ist ausserordentlich überraschend und war nicht vorauszusehen.

Neben Ethanol, das bei dem erfindungsgemässen Verfahren in hoher Selektivität entsteht, bilden sich in geringerem Mass weitere sauerstoffhaltige $C_2$-Produkte, wie Acetaldehyd und Essigsäure, ferner solche Produkte, die in einer Folgereaktion, z.B. durch Veresterung, Acetalisierung oder Kondensation aus diesen Produkten gebildet werden können.

Hierzu zählen u.a. Ethylacetat und das Diethylacetal des Acetaldehyds. Der Anteil an anderen sauerstoffhaltigen Verbindungen mit drei oder mehr Kohlenstoffatomen im Molekül ist sehr gering und liegt normalerweise unter 5 Mol.-% bezogen auf umgesetztes Kohlenmonoxid. Die Gesamtselektivität zu sauerstoffhaltigen $C_2$-Verbindungen, einschliesslich der in Ethylacetat und Acetaldehyddiethylacetal umgewandelten Produkte, beträgt bis zu 81%, bezogen auf umgesetztes Kohlenmonoxid; Das restliche Kohlenmonoxid wird, ausser zu den genannten Produkten mit 3 und mehr Kohlenstoffatomen, im wesentlichen zu Methan und anderen gasförmigen Kohlenwasserstoffen und im geringen Masse zu Kohlendioxid umgesetzt.

Zum Aufbau des Katalysators für das erfindungsgemässe Verfahren kann man von Salzen oder Komplexverbindungen des Rhodiums ausgehen. Geeignet sind z.B. Chloride, Bromide und Jodide des Rhodiums oder auch Doppelsalze des

Rhodiums mit Alkalihalogeniden, wie z.B. Dikaliumtrichlororhodat. Geeignet sind ferner Komplexverbindungen, die neben Rhodium und Halogen noch komplexbildende Liganden, wie Trialkylphosphine, Triarylphosphine, Ethylendiamin, Pyridin, Kohlenmonoxid, Olefine oder Wasser enthalten, also z.B. Tristriphenylphosphin-rhodium-I-chlorid, -bromid oder -jodid, Tris-triphenylphosphin -rhodium-III-chlorid, Dichlor-bis-ethylendiamin-rhodium-I-chlorid, Tris-ethylendiamin-rhodium-III-chlorid, Bis-tri-o-tolyl-phosphin-rhodium-II-chlorid, Carbonyl-bis-triphenyl-phosphin-rhodium-I-bormid oder Dicäsium-carbonyl-pentachloro-rhodat-III. Darüber hinaus kommen auch solche Verbindungen des Rhodiums in Betracht, in denen es ionogen oder komplex an einen Träger gebunden ist. Beispiele hierfür sind die mit Rhodiumhalogeniden ausgetauschten Zeolithe und Ionenaustauscher.

Als Cokatalysatoren werden erfindungsgemäss eines oder mehrere der Elemente Zirkon, Hafnium, Lanthan, Platin, Chrom und Quecksilber in Form ihrer Salze oder Komplexverbindungen auf den Träger aufgebracht. Vorzugsweise verwendet man Zirkon, Hafnium, Lanthan, Chrom oder Quecksilber, insbesondere Hafnium, Chrom oder Quecksilber. Es eignen sich einfache anorganische und organische Salze der genannten Elemente, wie z.B. die Chloride, Bromide, Nitrate, Formiate, Acetate. Vorzugsweise verwendet man die Chloride. Ferner können die Oxide, die Hydroxide oder die Carbonate verwendet werden, wenn man sie durch Behandlung mit Mineralsäuren oder Carbonsäuren in die genannten Salze überführt. Als Komplexverbindungen eignen sich besonders Chloro-komplexe mit Rhodium der allgemeinen Formel $Me_m[RhCl_6]_n$, wobei Me für den Cokatalysator steht, wie z.B. im Falle des Chroms der Komplex $Cr[RhCl_6] \cdot 2H_2O$, den man durch Umsetzung von Chromchlorid mit Rhodiumchlorid in Essigsäure bei 100 °C erhält.

Derartige Komplexe können durch Imprägnierung auf einen Träger aufgezogen werden; es ist jedoch wegen der geringen Löslichkeit einiger Komplexe häufig vorteilhaft, den Träger mit einer essigsauren Lösung von Rhodium-III-chlorid und einem oder mehreren Chloriden der als Cokatalysatoren genannten Elemente zu imprägnieren und ihn anschliessend einer Temperatur von 100 °C auszusetzen, wobei sich in den Trägerporen die Komplexe bilden. Jedoch kann das cokatalytisch wirksame Element auch vorher auf den Träger aufgebracht oder auch in eine Gerüstsubstanz eingebaut sein, beispielsweise in eine Silikat- oder Aluminiumoxid enthaltende Trägersubstanz, wie Kieselsäure, Aluminiumoxid oder Aluminiumsilikat. Eine weitere vorteilhafte Möglichkeit besteht darin, die Kationen der Elemente mittels Ionenaustausch an Kationenaustauscher zu binden, die auch als Träger für das Rhodium geeignet und unter den Versuchsbedingungen beständig sind, beispielsweise die als Molsiebe bekannten natürlichen oder synthetischen Aluminiumsilikate. Es führt aber auch die umgekehrte Reihenfolge der Trägerimprägnierung, d.h. zunächst die Imprägnierung mit Rhodiumverbindungen und dann die mit den jeweiligen Cokatalysatoren zu geeigneten Katalysatoren. Die Leistung – bei unverändert hoher Selektivität zu Ethanol – kann durch Zusatz weiterer Promotoren, insbesondere Magnesium, noch erhöht werden.

Als Katalysatorträger können übliche Trägermaterialien mit unterschiedlichen spezifischen Oberflächen verwendet werden. Allerdings werden Träger mit spezifischen Oberflächen von 50 bis 1000 $m^2/g$ bevorzugt. Geeignet sind z.B. Kieselsäure, natürliche oder synthetische Silikate von Elementen der II. bis VIII. Gruppe des Periodischen Systems (also beispielsweise die Silikate des Magnesiums, Calciums, Aluminiums, Mangans), ferner Aluminiumoxid, Thoriumdioxid, Zeolithe und Spinelle. Vorzugsweise verwendet man Kieselsäure oder Silikate.

Zur Herstellung der Katalysatoren werden die Träger mit den aktiven Komponenten gleichzeitig oder in aufeinanderfolgenden Stufen getränkt. Bei Einsatz von Rhodium-III-salzen ist die nachfolgende Behandlung mit geeigneten Reduktionsmitteln, wie Wasserstoff, Kohlenmonoxid, Methanol vorteilhaft. Diese Reduktion kann in einer getrennten Apparatur oder im Reaktor selbst durchgeführt werden. Im allgemeinen sind hierfür Temperaturen unter 300 °C, vorzugsweise zwischen 100 und 275 °C, geeignet. Vielfach ist es zweckmässig, die Reduktion nicht mit den unverdünnten reduzierend wirkenden Gasen, sondern mit einem zusätzlichen Anteil an Inertgasen, wie z.B. Stickstoff, Kohlendioxid oder auch Edelgasen, vorzunehmen.

Es können aber auch die Träger in Gegenwart der aktiven Komponenten erst hergestellt werden, z.B. durch gemeinsame Fällung der aktiven Komponenten mit Silikaten.

Die Konzentration an Rhodium und Cokatalysator in den Katalysatoren kann in weiten Grenzen variiert werden; im allgemeinen liegen die Werte zwischen 0,1 und 20 Gew.-% für Rhodium, und zwischen 0,1 und 25 Gew.-% für die Cokatalysatoren. Bevorzugt sind Katalysatoren mit 1,0 bis 10 Gew.-% Rhodium und 0,1 bis 20 Gew.-% Cokatalysator.

Zur Durchführung des erfindungsgemässen Verfahrens werden Gasgemische, die ganz oder zu einem überwiegenden Teil aus Kohlenmonoxid und Wasserstoff bestehen und daneben gegebenenfalls noch andere Komponenten wie Stickstoff, Argon, Kohlendioxid oder Methan enthalten können, über den Katalysator geleitet. Das molare Verhältnis von Kohlenmonoxid zu Wasserstoff kann dabei in weiten Grenzen variiert werden. Bevorzugt sind Molverhältnisse zwischen 5 : 1 und 1 :5 und besonders zwischen 3 : 1 und 1 : 3. Die Reaktionstemperaturen liegen im allgemeinen zwischen 175 und 375 °C, vorzugsweise zwischen 200 und 350 °C, und die Reaktionsdrücke zwischen 1 und 300 bar, vorzugsweise zwischen 20 und 200 bar.

Zweckmässig ist es, Temperatur und Druck so aufeinander abzustimmen, dass eine hohe Selektivität zu den sauerstoffhaltigen Verbindungen gewährleistet ist und die bei höheren Temperaturen begünstigte exotherme Bildung von Methan gering gehalten wird. Man wird deshalb hohe Drükke und möglichst niedrige Temperaturen bevorzugen. Der Umsatz an Kohlenmonoxid sollte dabei im allgemeinen nicht über 50% liegen, da höhere Umsätze leicht zu vermehrter Nebenproduktbildung führen können, wobei neben Methan, Kohlendioxid und gasförmigen Kohlenwasserstoffen auch höhermolekulare flüssige Kohlenwasserstoffe und sauerstoffhaltige Produkt auftreten können.

Für die Verfahrensdurchführung ist die Gasphase bevorzugt. Hierzu können die herkömmlichen Festbettreaktoren verwendet werden, wobei es zur besseren Wärmeabführung vorteilhaft ist, die Katalysatorschichtdicke gering zu halten. Ferner sind auch Reaktoren mit bewegtem Katalysatorbett oder Wirbelbettreaktoren geeignet.

Man kann aber auch eine Umsetzung des Synthesegases in Gegenwart des festen und feinverteilten Katalysators, suspendiert in inerten Lösungsmitteln und/oder Reaktionsprodukten, durchführen.

Eine besonders bevorzugte Ausführungsform der Erfindung besteht darin, die Umsetzung in einer Kreisgasapparatur in der Gasphase durchzuführen, in der nach Abtrennung der kondensierbaren Reaktionsprodukte das nicht umgesetzte Gasgemisch wieder in den Reaktor zurückgeführt wird.

Diese Verfahrensweise ist besonders wirtschaftlich und ermöglich durch Verdünnung des Frischgases mit dem im Kreislauf zurückgeführten wasserstoffärmeren Restgas höhere Reaktionstemperaturen und damit höhere Raum-Zeit-Ausbeuten bei unveränderten Selektivitäten. Als Kreisgasapparaturen können dabei solche mit innerem oder äusserem Gasumlauf in Betracht kommen.

Die Erfindung soll durch die folgenden Beispiele erläutert werden, wobei die Beispiele aber in keiner Weise einschränkend sein sollen.

**Beispiele**

A) **Allgemeine Versuchsbeschreibung**

Die Apparatur besteht aus einem beheizten Reaktionsrohr von 1 m Länge und 16 mm innerem Durchmesser aus korrosionsbeständigem Stahl mit einer koaxial angebrachten Thermometerhülse von 6 mm äusserem Durchmesser, einem nachgeschalteten Kondensator, einer Vorlage für das Kondensat und einem Kompressor für die Rückführung eines Teils der nichtkondensierten Gase zum Reaktor (Kreisgas). Es werden jeweils 100 ml der unten beschriebenen Katalysatoren eingefüllt. Nach Spülen der Apparatur mit Stickstoff wird zunächst mit einem Synthesegas der Zusammensetzung 49 Vol.-% CO, 49 Vol-% $H_2$, 1 Vol.-% $CO_2$, 1 Vol-% $N_2$ (und geringe Mengen anderer Komponenten) ein Druck von 100 bar eingestellt und der Reaktor auf 275 °C aufgeheizt. Während des Aufheizens und im weiteren Versuchsverlauf werden stündlich 450 Nl Synthesegas der obigen Zusammensetzung über die Saugseite des Kompressors dem Kreisgas zugeführt und zusammen mit diesem über den Katalysator geleitet. Das den Reaktor verlassende Gasgemisch wird in dem solegekühlten Kondensator auf etwa +5 °C abgekühlt und die kondensierten Anteile in der Vorlage aufgefangen. Das nicht kondensierte Restgas wird nach Vermischen mit frischem Synthesegas über den Kompressor wieder dem Reaktor zugeführt. Zur Aufrechterhaltung des Drucks und zur Ausschleusung von Nebenprodukten wird ein Teil des Restgases über ein Druckhalteventil als Abgas abgeleitet. Nach dieser Methode werden die nachstehend beschriebenen Katalysatoren geprüft. In der Tabelle sind die Laufzeit der Versuche, die Raum-Zeit-Ausbeuten an sauerstoffhaltigen $C_2$-Produkten pro Liter Katalysator und Stunde sowie die Selektivitäten zu Ethanol, Acetaldehyd und Essigsäure (in Mol-% CO, bezogen auf umgesetztes CO) zusammengestellt. Geringe Mengen an entstandenem Ethylacetat bzw. Acetaldehyddiethylacetal werden in Essigsäure, Ethanol bzw. Acetaldehyd umgerechnet.

B) **Herstellung der Katalysatoren**

Je 40 g Kieselsäure mit einer BET-Oberfläche von 270 $m^2/g$ einem Porenvolumen von 1,27 ml/g und einem Schüttgewicht von 0,4 kg/l werden mit einer Lösung von 5,2 g $RhCl_3 \cdot xH_2O$ (38,0 Gew.-% Rh) in 50 ml Wasser imprägniert und 1,5 Stunden bei 80 °C, 1,5 Stunden bei 110 °C und 1,5 Stunden bei 150 °C getrocknet.

Dieser Katalysator wird für das Vergleichsbeispiel verwendet.

Für die Beispiele 1 bis 6 wird der Katalysator jeweils noch mit einer wässrigen oder alkoholischen Lösung der folgenden Chloride in je 50 ml Lösungsmittel imprägniert und 2 Stunden bei 80 °C und dann 2 Stunden bei 150 °C getrocknet.

| Beispiel Nr. | Zusatz | Menge (g) |
|---|---|---|
| 1 | $Zr\,Cl_4$ | 3.4 |
| 2 | $La\,Cl_3$ | 4.8 |
| 3 | $Pt\,Cl_4$ | 4.9 |
| 4 | $Cr\,Cl_3 \cdot 6\,H_2O$ | 5.2 |
| 5 | $Hf\,Cl_4$ | 4.7 |
| 6 | $Hg\,Cl_2$ | 7.9 |

Darauf werden die Katalysatoren für die Beispiele 1 bis 6 noch im Glaskolben am Rückflusskühler mit 50 ml Essigsäure 5 Stunden auf 100 °C erhitzt und danach 1,5 Stunden bei 110 °C, dann 3 Stunden bei 150 °C und schliesslich 1 Stunde bei 300 °C unter Stickstoff getrocknet.

Die Reduktion der Katalysatoren für die Beispiele 1 bis 6 erfolgt in einem Strömungsrohr aus Glas durch 3-stündiges Überleiten von 30 Nl/h Wasserstoff bei 225–275 °C unter Normaldruck.

C) **Versuchsergebnisse**

In der nachfolgenden Tabelle sind die mit den Katalysatoren erhaltenen Versuchsergebnisse zusammengestellt. Es sind Durchschnittswerte über Versuchszeiten von je 100 Stunden:

**Tabelle**

Reaktionsbedingungen: Kreisgasapparatur, 100 bar, 275 °C, Einsatzgas 400 Nl/h, mit Verhältnis CO : $H_2$ = 1 : 1, Katalysatorvolumen 0,1 l, Versuchszeit 100 Stunden.
(AcOH = Essigsäure, AcH = Acetaldehyd, EtOH = Ethanol)

| Beispiel Nr. | Katalysator | RZA in g/l·h[1)] | | Selektivität (mol.% CO)[2)] | | | |
|---|---|---|---|---|---|---|---|
| | | $\Sigma C_2$–O | EtOH | AcOH | AcH | EtOH | $\Sigma C_2$–O |
| Vergleichsbeispiele | Rh | 52 | 31 | 17,2 | 6,4 | 24,4 | 48,0 |
| 1 | Rh/Zr | 390 | 343 | 4,1 | 1,8 | 70,1 | 76,0 |
| 2 | Rh/La | 380 | 318 | 7,0 | 4,0 | 67,5 | 78,5 |
| 3 | Rh/Pt | 350 | 320 | 3,2 | 2,4 | 75,0 | 80,6 |
| 4 | Rh/Cr | 390 | 351 | 5,4 | 3,5 | 68,1 | 77,0 |
| 5 | Rh/Hf | 360 | 311 | 6,4 | 2,2 | 66,4 | 75,0 |
| 6 | Rh/Hg | 375 | 340 | 4,0 | 2,5 | 74,5 | 81,0 |

[1)]RZA = Raum-Zeit-Ausbeute in Gramm pro Liter Katalysator und Stunde zu den sauerstoffhaltigen $C_2$-Verbindungen Essigsäure, Acetaldehyd und Ethanol ($\Sigma C_2$–O) bzw. zu Ethanol allein (EtOH).

[2)]Molprozent bezogen auf umgesetztes Kohlenoxid.

**Patentansprüche**

1. Verfahren zur Herstellung von Ethanol durch Umsetzung von Kohlenmonoxid mit Wasserstoff an einem Rhodium-Trägerkatalysator, dadurch gekennzeichnet, dass der Trägerkatalysator eines oder mehrere der Elemente Zirkon, Hafnium, Lanthan, Platin, Chrom und Quecksilber in Form ihrer Salze oder Komplexverbindungen als Cokatalysator, aber im Falle von Zirkon und Chrom kein Magnesium enthält.

2. Verfahren nach Anspruch 1, dadurch gekennzeichnet, dass man als Katalysator-Träger Kieselsäure oder Silikate verwendet.

3. Verfahren zur Herstellung von Ethanol durch Umsetzung von Kohlenmonoxid mit Wasserstoff an einem Rhodium-Trägerkatalysator, dadurch gekennzeichnet, dass man Rhodium in Form chlorhaltiger Komplexe der allgemeinen Formel $Me_m[RhCl_6]_n$, wobei Me für eines der katalytisch wirkenden Elemente Zirkon, Hafnium, Lanthan, Platin, Chrom und Quecksilber steht, verwendet.

**Revendications**

1. Procédé de préparation de l'éthanol par réaction du monoxyde de carbone avec l'hydrogène en présence d'un catalyseur constitué de rhodium déposé sur un support, procédé caracterisé en ce que le catalisateur sur support contient, en tant que co-catalyseur, un ou plusieurs des éléments zirconium, hafnium, lanthane, platine, chrome et mercure sous la forme de sels ou de complexes, et, dans le cas du zirconium et du chrome, ne contient pas de magnésium.

2. Procédé selon la revendication 1 caractérisé en ce qu'on utilise, comme support du catalyseur, de la silice ou des silicates.

3. Procédé de préparation de l'éthanol par réaction du monoxyde de carbone avec l'hydrogène en présence d'un catalyseur constitué de rhodium déposé sur un support, procédé caractérisé en ce qu'on utilise le rhodium sous la forme de complexes chlorés répondant à la formule générale:

$$Me_m[RhCl_6]_n$$

dans laquelle Me représente l'un des éléments à action catalytique suivants: zirconium, hafnium, lanthane, platine, chrome et mercure.

**Claims**

1. Process for the manufacture of ethanol by reaction of carbon monoxide with hydrogen in the presence of a supported rhodium catalyst, characterized in that the supported catalyst contains at least one of the elements zirconium, hafnium, lanthanum, platinum, chromium and mercury in the form of their salts or complex compounds as co-catalyst, but zirconium or chromium only in the absence of magnesium.

2. The process of claim 1, wherein the catalyst is supported on silicic acid or on a silicate.

3. Process for the manufacture of ethanol by reaction of carbon monoxide with hydrogen in the presence of a supported rhodium catalyst, characterized in that rhodium is used in the form of a chlorine-containing complex compound of the formula $M_m$ $(RhCl_6)_n$ in which M denotes one of the co-catalytic elements zirconium, hafnium, lanthanum, platinum, chromium and mercury.